# EUROPÄISCHE PATENTSCHRIFT

(11) **EP 1 225 953 B1**
(45) Veröffentlichungstag und Bekanntmachung des Hinweises auf die Patenterteilung: **07.11.2007**
(21) Anmeldenummer: 00960456.2
(22) Anmeldetag: 16.08.2000
(51) Int. Cl.: A61N 1/06, A61B 18/14

(54) **ELEKTRODENKATHETER MIT REDUZIERTER STÖRAKTIVITÄT SOWIE DIESBEZÜGLICHES BEHANDLUNGSVERFAHREN DER ELEKTRODENOBERFLÄCHE**
ELECTRODE CATHETER WITH REDUCED NOISE ACTIVITY AS WELL AS A RELATED PROCESSING METHOD FOR THE ELECTRODE SURFACE
CATHETER A ELECTRODES A ACTIVITE PERTURBATRICE REDUITE ET PROCEDE DE TRAITEMENT DE LA SURFACE DES ELECTRODES APPROPRIE

(30) Priorität: 17.08.1999 DE 19938558
(43) Veröffentlichungstag der Anmeldung: 31.07.2002
(73) Patentinhaber: Muntermann, Axel, 35578 Wetzlar (DE)
(72) Erfinder: Muntermann, Axel, 35578 Wetzlar (DE)
(74) Vertreter: Herden, Andreas F.
(86) Internationale Anmeldenummer: PCT/EP2000/007943
(87) Internationale Veröffentlichungsnummer: WO 2001/012259

(56) Entgegenhaltungen:
- DE-A- 19 740 976
- US-A- 4 653 500
- US-A- 5 810 764

## Beschreibung

Die Erfindung betrifft einen Katheter und ein Verfahren zur Austellung von Kathetern.

Bei der Katheterablation myokardialen Gewebes ist es eines der Hauptziele, durch Läsionen der oberen Schichten des Herzgewebes Reizleitungsbereiche zu unterbrechen, welche die Herztätigkeit negativ beeinflussen können. Der Erfolg einer Behandlung hängt jedoch ganz wesentlich davon ab, ob bei der Ablation die richtige Läsionstiefe erreicht wurde. Richtige Läsionstiefe bedeutet hierbei im wesentlichen, daß die unerwünschten, die Reizleitung störenden Bereiche ausgeräumt, aber keine darüber hinaus gehenden Schädigungen eingebracht wurden. Es ist offensichtlich, daß bei zu geringer Läsionstiefe der Behandlungserfolg gefährdet ist, eine zu große Tiefe erzeugt jedoch unter Umständen sehr viel schwerwiegendere Nebenwirkungen. Da im Herzen Gefäßwände verlaufen, die nicht unnötig geschädigt werden dürfen und auch das zu abladierende Gewebe häufig nur eine begrenzte Dicke aufweist, können bei zu großen Tiefen der Läsionen sogar letale Unfälle aufgrund durchtrennter Herzwände oder - gefäße auftreten. Bei herkömmlichen Ablationsverfahren wurde folglich versucht, durch die synchrone Aufzeichnung von EKG-Signalen am eintretenden Behandlungserfolg die optimale Läsionstiefe zu ermessen. Hierbei war jedoch die eingestrahlte Hochfrequenzenergie der Aufzeichnung dieser Signale äußerst abträglich und es wurde der Versuch unternommen, derartige Einflüsse durch entsprechende elektrische oder elektronische Filter in den nachgeschalteten Geräten zu mildern. Diese Versuche waren jedoch nur begrenzt oder nicht erfolgreich. Die Verminderung der eingestrahlten Leistung führte zu extrem langen Behandlungszeiten, welche im Bereich mehrerer Stunden liegen und hierbei sowohl den Patienten erheblich belasten als auch ein Verrutschen des Ablationskatheters nicht sicher vermeiden können. Ferner ist ab einer bestimmten Leistung keine Läsion mehr möglich, da die erzeugte Temperatur für eine Gewebekoagulation nicht mehr ausreicht. Das Dokument US-A-5 810 764 offenbart eine Vorrichtung gemäß Oberbegriff des Anspruch 1.

Folglich liegt der Erfindung die Aufgabe zu Grunde, die Aufzeichnung von EKG-Signalen bei der Katheterablation zu ermöglichen und insbesondere die Qualität der aufgezeichneten EKG-Signale soweit zu verbessern, daß medizinische Aussagen in Bezug auf die Herztätigkeit ermöglicht werden.

Diese Aufgabe wurde durch die Erfindung in höchst überraschender Weise mit einem Katheter nach Anspruch 1 bzw. einem Verfahren zur Katheterherstellung nach Anspruch 9 gelöst.

Der Erfinder ging in überraschender Weise einen vollständig anderen Weg als dies beim bekannten Stand der Technik bisher getan wurde.

Es wurden nicht die Geräte zur Aufzeichnung verändert bzw. versucht zu verbessern sondern es wurde die Ursache der Störungen der Aufzeichnung der EKG-Signale vermindert oder sogar vollständig beseitigt.

Der Erfinder fand erstmalig heraus, daß die Ursache der elektrischen Störungen der EKG-Aufzeichnung bei simultaner Einstrahlung von Hochfrequenzenergie im wesentlichen nicht in den Leitungen zu und von den Katheterelektroden, nicht in den elektronischen Aufzeichnungseinrichtungen und insbesondere nicht in deren Eingangsfiltern liegen sondern in elektrische Störzentren im Bereich der Oberfläche der Ablations- oder Mappingelektroden liegt.

Diese Erkenntnis war um so überraschender als jeglicher untersuchter Ablationskatheter mit Platinelektroden derartige elektrische Störzentren aufwies und nach deren Verminderung oder Entfernung nahezu oder vollständig frei von den vorstehend beschriebenen unerwünschten Störungen war.

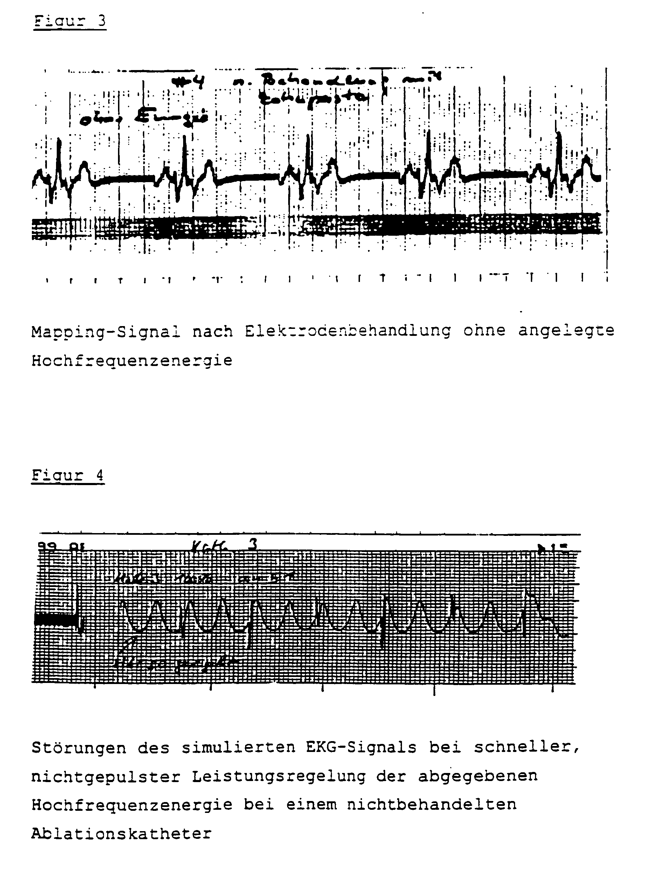

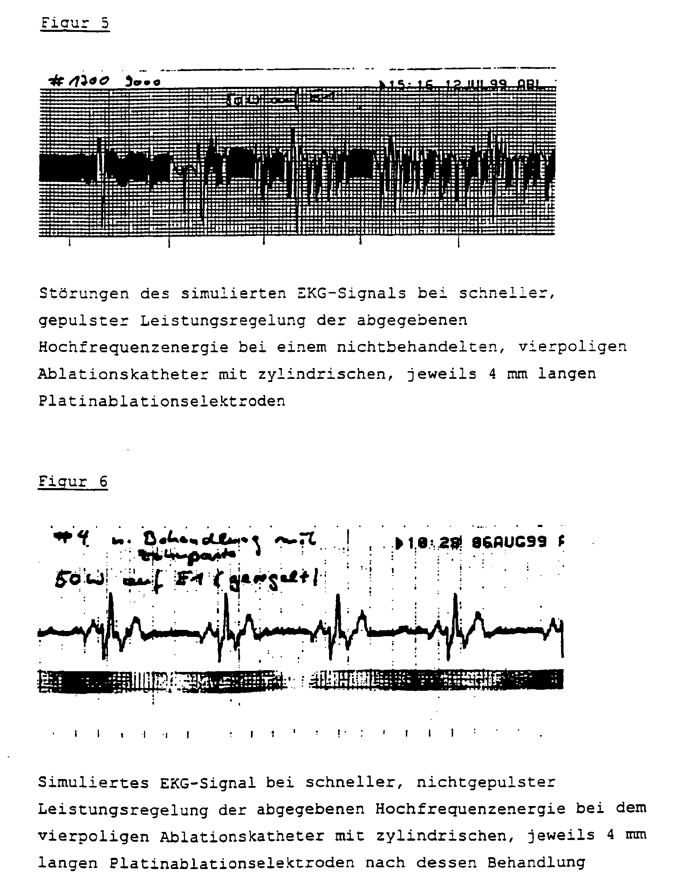

Gemäß der Erfindung weist bei einem Katheter zur Ablation von biologischem, insbesondere von tierischem oder menschlichem Gewebe, vorzugsweise zur Ablation von myokardialem Gewebe des Menschen, mit mindestens einer Ablations- oder Mappingelektrode diese mindestens eine Ablations- oder Mappingelektrode eine reduzierte Anzahl elektrischer Sörzentren auf. Hierdurch werden beispielsweise die in Fig. 4, 5 und 7 dargestellten, gestörten EKG-Aufzeichnungen derart verbessert, daß die in Fig. 6 dargestellten Signale erhalten werden können.

In besonders vorteilhafter Weise sind die elektrischen Störzentren, welche bei Abgabe von Hochfrequenzenergie an der zumindest einen Ablations- oder Mappingelektrode elektrische Signale erzeugen und die im wesentlichen an Oberflächenbereichen der zumindest einen Ablations- oder Mappingelektrode angeordnet sind in deren Anzahl, flächigen Erstreckung und/oder elektrischen Wirkung vermindert.

Hierdurch kommt es zu einer Entfernung oder elektrischen Deaktivierung des Einfluß dieser Störzentren.

Ein besonders wirkungsvolles Verfahren, um die vorstehenden Erfolge zu erzielen besteht darin, daß die zumindest eine Ablations- oder Mappingelektrode eine mechanisch behandelte Oberfläche aufweist, da hierdurch in der Oberfläche liegende elektrische Störzentren abgetragen und/oder in deren Wirkung vermindert werden können.

Bevorzugt weist die zumindest eine Ablations- oder Mappingelektrode eine mechanisch behandelte Oberfläche auf, die mit einem die Oberfläche glättenden Verfahren behandelt ist. Bisherige Oberflächenbearbeitungen, beispielsweise bei Platin-Iridium-Kathetern zielten darauf ab, die Oberfläche zu vergrößern, d.h. gerade nicht zu glätten sondern rauhe Strukturen mit einer um etwa den Faktor 1000 größerer Oberfläche zu schaffen; die Erfindung geht jedoch überraschend erfolgreich gerade den entgegengesetzten Weg.

Bei einer besonders bevorzugten und vorteilhaften Ausführungsform ist vorgesehen, daß die zumindest eine Ablations- oder Mappingelektrode ein Edelmetall, insbesondere Platin umfaßt, dessen Oberfläche geglättet, insbesondere geläppt oder poliert ist. Es hat sich hierbei als besonders effektiv herausgestellt, eine Schleif- oder Poliermittel zu verwenden, welches Kalziumkarbonat enthält.

Alternativ oder zusätzlich kann vorgesehen sein, daß die Ablations- oder Mappingelektrodenoberfläche mit einer glatten Walze gewälzt ist.

Nach der vorstehend beschriebenen Behandlung war häufig zu beobachten, daß Strukturen der Oberfläche der mindestens eine Ablations- oder Mappingelektrode eine verrundete Oberflächenstruktur aufweisen, deren Kanten einen Radius von mehr als ungefähr 500 nm, vorzugsweise von mehr als 100 nm aber wenigstens mehr als 10 nm aufweisen und es wird vermutet, daß diese Oberflächenveränderungen bereits zumindest einen Teil der Verminderung der elektrischen Störzentren oder deren Wirkungen hervorrufen.

Ferner wird angenommen daß durch die mechanische Behandlung auch an der Oberfläche vorhandene elektrische Feldstärkespitzen, beispielsweise durch Korngrenzen des kristallin vorliegenden Metalls hervorgerufen, geglättet bzw. kompensiert werden und nach der erfindungsgemäßen Behandlung auch mikroskopische elektrische Feldstärkedifferenzen oder mikroskopisch unterschiedliche Reaktiosvermögen an der Elektrodenoberfläche gemildert oder ausgeglichen sind. Hierdurch werden die bei der HF-Energieabgabe auftretenden Phänomene, die ohne Beschränkung der Allgemeinheit oder der Breite der Erfindung, lokal verschiedener ionischer Beweglichkeit zugeschrieben werden, gemildert, denn es findet kein "Nachlaufen" von durch verschiedene Feldstärken stärker oder schwächer bewegten Ionen oder polaren Molekülen mehr statt, durch welches störende elektrische Potentiale oder Feldstärken gebildet werden, die sich dem EKG-Signal als Störsignal überlagern. Die sich nun an allen Orten der Oberfläche der Ablations- oder Mappingelektrode nahezu gleich bewegenden Ionen erzeugen keine lokalen Potential- oder Feldstärkedifferenzen mehr und stören auch nicht mehr die EKG-Aufzeichnung.

Folglich wird davon ausgegangen, daß wenn der Katheter in vorteilhafter Weise eine Platin-Ablations- oder Mappingelektrode umfasst, die Oberfläche der zumindest einen Ablations- oder Mappingelektrode zumindest bereichsweise mit elementarem Platin belegt oder versehen ist. Es liegt im Rahmen der Erfindung, eine derartige atomare, im wesentlichen nichtkristalline oder amorphe Belegung aber auch beispielsweise mit Auftraguns- oder Plattierungstechniken, wie beispielsweise dem Aufreiben dünner, vorzugsweise amorpher Folien zu erzeugen.

In vorteilhafter Weise ergibt es sich dann, daß die Oberfläche der zumindest einen Ablations- oder Mappingelektrode Bereiche mit abgeschiedenem, im wesentlichen amorph oder atomar vorliegendem Metall umfasst.

Die Erfindung wird nachfolgend anhand bevorzugter Ausführungsformen und unter Bezugnahme auf die beigefügten Zeichnungen detaillierter erläutert.

Es zeigen:
- Figur 1: eine schematische Darstellung einer Vorrichtung zur Messung von simulierten EKG-Signalen mit und ohne eingestrahlte Hochfrequenzenergie,
- Figur 2: ein simuliertes EKG-Signal, als Mapping-Signal, vor Elektrodenbehandlung ohne angelegte Hochfrequenzenergie,
- Figur 3: ein simuliertes EKG Signal, als Mapping-Signal, nach Elektrodenbehandlung ohne angelegte Hochfrequenzenergie,
- Figur 4: Störungen des simulierten EKG-Signals bei schneller, nichtgepulster Leistungsregelung der abgegebenen Hochfrequenzenergie bei einem nichtbehandelten Ablationskatheter,
- Figur 5: Störungen des simulierten EKG-Signals bei schneller, gepulster Leistungsregelung der abgegebenen Hochfrequenzenergie bei einem nichtbehandelten, vierpoligen Ablationskatheter mit zylindrischen, jeweils 4 mm langen Platinablationselektroden,
- Figur 6: ein simuliertes EKG-Signal bei abgegebener Hochfrequenzenergie bei dem vierpoligen Ablationskatheter mit zylindrischen, jeweils 4 mm langen Platinablationselektroden nach dessen Behandlung,
- Figur 7: Störungen des simulierten EKG-Signals bei schneller, gepulster Leistungsregelung der abgegebenen Hochfrequenzenergie bei einem nichtbehandelten, Ablationskatheter mit einer zylindrischen, 4 mm langen Platinablationselektrode und drei weiteren Mappingelektroden,
- Figur 8: eine elektronenmikroskopische Aufnahme der Platinoberfläche der Ablationselektrode eines nichtbehandelten Ablationskatheters in 1960 facher Vergrößerung
- Figur 9: eine elektronenmikroskopische Aufnahme der Platinoberfläche der Ablationselektrode des nichtbehandelten Ablationskatheters aus Figur 10 in 6160 facher Vergrößerung
- Figur 10: eine AFM- (atomic force microscopic) bzw. kraftmikroskopische Aufzeichnung eines 10 mal 10 µm großen Oberflächenbreichs einer unbehandelten Platin-Ablationselektrode.

Die Erfindung wird nachfolgend detaillierter und unter Bezugnahme auf die beigefügten Zeichnungen beschrieben.

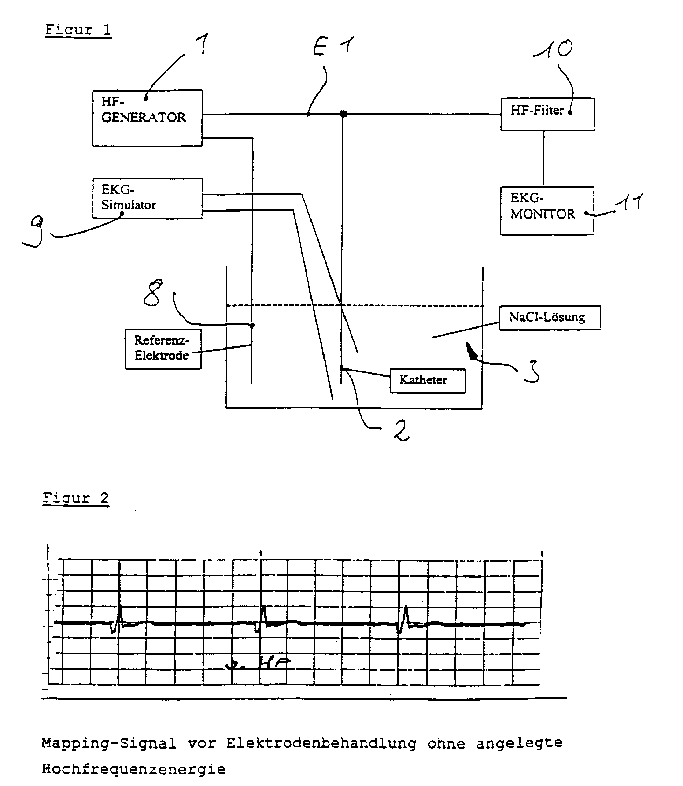

Zunächst wird auf Fig. 1 Bezug genommen, welcher eine schematische Darstellung einer Vorrichtung zur Messung von simulierten EKG-Signalen mit und ohne eingestrahlte Hochfrequenzenergie zu entnehmen ist, welche sowohl zur Aufzeichnung der gestörten EKG-Signale mit unbehandelten Kathetern als auch zur Aufzeichnung der verbesserten Signale mit behandelten Kathetern verwendet wurde.

Fig. 1 zeigt einen Hochfrequenz-Generator 1, der mit einem Katheter 2 verbunden ist sowie ein elektrolytbefülltes Gefäß 3.

Der Katheter 2 ist bei dem Beispiel aus Fig. 1 mit zumindest einer Ablations- oder Mappingelektrode, die über eine Zuleitung E1 mit dem Generator 1 verbunden ist, versehen sowie mit einer oder mehreren weiteren Elektroden, welche über eine oder mehrere weitere Zuleitungen mit dem Generator 1 verbunden ist/sind. Die weiteren Elektroden können Mapping- oder eine oder mehrere Ablationselektroden sein.

Das Gefäß 6 war mit physiologischer NaCl-Lösung befüllt und der Katheter 2 derart darin angeordnet, daß dessen Ablations- oder Mappingelektrode vollständig von der NaC1-Lösung benetzt war. Ferner war der Katheter 2 an den herkömmlichen Hochf-requenzgenerator 7 so angeschlossen, daß die bei der Ablation typischen Hochfrequenzenergiewerte der Ablationselektrode des Katheters 2 zugeführt wurden.

Das HF-Feld wurde von dem HF-Generator 1 zwischen der Ablations- oder Mappingelektrode des Katheters 2 und einer Referenzelektrode 8 erzeugt, und stellte auf diese Weise in sehr guter Nährung eine Situation dar, wie sie auch beispielsweise im menschlichen Herzen vorliegt.

Mit einem EKG-Simulator 9 wurden Spannungssignale erzeugt, welche in sehr guter Näherung, den vom menschlichen Herzen abgegebenen elektrischen Spannungen sowohl von der Höhe als auch von deren zeitlichem Verlauf entsprachen.

Der Katheter 2 war ferner an ein Hochfrequenzfilter 10 angeschlossen, welcher die vom HF-Generator 1 eingespeisten Hochfrequenz-Signalanteile ausfilterte. Derartige Filteranordnungen sind dem Fachmann wohl bekannt und können beispielsweise den im QuadraPulse-Gerät der AD-Elektronik verwendeten Eingangsfilter entsprechen.

Das vom Katheter abgenommene, insbesondere von dessen Mapping-Elektrode, oder sogar dessen Ablationselektrode gewonnene EKG-Signal wurde dann einem EKG-Monitor 11 zugeführt, wie dieser beispielsweise von der Firma Physiocontrol unter der Bezeichnung LIFEPAK 10 oder von der Firma Bard als EP-Laborsystem vertrieben wird.

Der Katheter 2 wurde nach dessen Behandlung gemessen und es stellte sich heraus, daß die zumindest eine Ablations- oder Mappingelektrode mit mechanisch behandelter Oberfläche weniger elektrische Störzentren aufweist.

Die mechanisch behandelte Oberfläche wurde gemäß der Erfindung mit einem die Oberfläche glättenden Verfahren behandelt, bei welchem Strukturen der Oberfläche der mindestens einen Ablations- oder Mappingelektrode eine verrundete Oberflächenstruktur aufweisen, deren Kanten einen Radius von mehr als ungefähr 500 nm, vorzugsweise von mehr als 100 nm aber wenigstens mehr als 10 nm aufweisen.

Diese Verrundung konnte beispielsweise dadurch erzeugt werden, daß die Ablations- oder Mappingelektrodenoberfläche mit einem Schleif- oder Poliermittel poliert ist, welches Kalziumkarbonat enthält geläppt oder poliert wurde. Generell wird erfindungsgemß mit einem weichen Schleif- oder Poliermittel behandelt, welches ein Karbonat, vorzugsweise ein Alkali- oder Erdalkalikarbonat umfasst. In überraschender Weise zeigten sich Poliermittel der Munhygiene, wie beispielsweise Zahnpasta oder Zahnpolier und - reinigungspräparate in vielen Fällen als gut geeignet.

Alternativ oder zusätzlich wurde die Ablations- oder Mappingelektrodenoberfläche mit einer glatten Walze gewälzt, um hierdurch ebenfalls zu der erwünschten mikroskopischen Glättung zu gelangen.

Ferner umfasst die mechanische Bearbeitung alternativ oder zusätzlich das Auftragen einer Folie mit glatter Oberfläche, insbesondere das Aufreiben der Folie mit glatter Oberfläche auf die zumindest eine Ablations- oder Mappingelektrode, sodaß die glatte Folienoberfläche mit der äußeren Umgebung in Wechselwirkung tritt.

Als Katheter eignen sich für die Durchführung der Erfindung im wesentlichen alle bekannten Ablationskatheter, insbesondere Katheter mit Platinelektroden, und es wurden bei den Untersuchungen des Erfinders beispielsweise die nachfolgend aufgeführten Katheter erfolgreich verwendet:
- 1.: BARD Side Winder Catheter S/N: 17009000
- 2.: BARD SideWinder Catheter S/N: 1300013000
- 3.: Cordis Webster Catheter Intemal S/N: CW1
- 4.: Cardiac Pathways Catheter S/N: G709313
- 5.: Biotronic Catheter: AICath Twin (nicht Ablations Katheter, Fraktale Pt/Ir-Oberfläche)
- 6.: BARD Stinger Distal Tip Ablationskatheter 4mm Tip
- 7.: BARD Stinger Distal Tip Ablationskatheter 8mm Tip
- 8.: Biotronic Catheter AlFractal, Distal Tip Ablationskatheter (Fraktale Pt/Ir-Oberfläche)

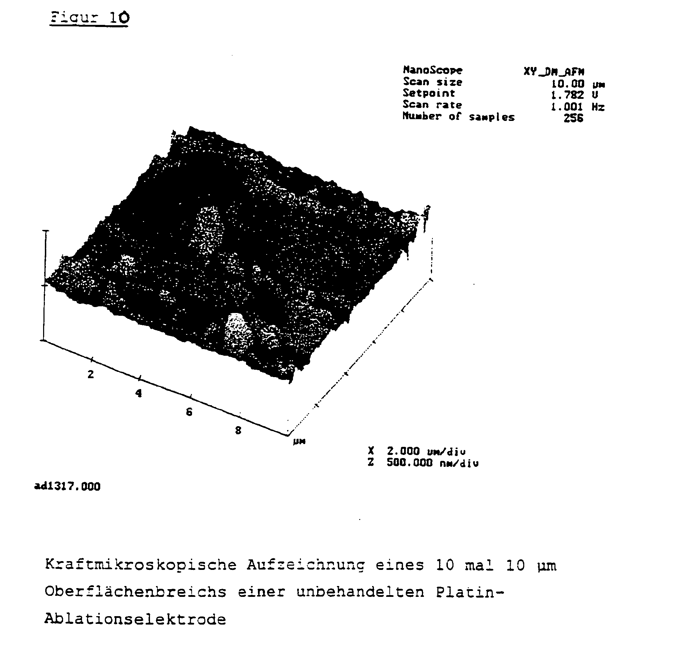

Die erhaltenen Ergebnisse werden nachfolgend unter Bezug auf die Figuren 2 bis 10 detaillierter erläutert.

Solange den Katheterelektroden keine Hochfrequenz-Energie bzw. Hochfrequenzspannung zugeführt wurde, weisen die Figuren 2 und 3 nach, daß die Aufzeichnung der EKG-Signale nahezu ungestört vorgenommen werden konnten.

Regelt man jedoch während der EKG-Aufzeichnung die Höhe der Hochfrequenzspannung bzw. die Menge an eingestrahlter Hochfrequenz-Energie wie es während eine realen Ablationsvorgangs am Patienten der Fall ist, so kommt es zu Spannungen, welche nahezu linear proportional zur eingestrahlten Energie verlaufen und beispielsweise in Fig. 4 dargestellt sind.

Eine Regelung der abgegebenen Energie im Wege einer Leistungsregelung der eingestrahlten Hochfrequenz-Energie führt somit stets zu einer Störsignalüberlagerung der EKG-Signale, welche es dem Mediziner in der Regel unmöglich machen, eine Aussage über den Behandlungserfolg oder den aktuellen Zustand des Herzens zu treffen.

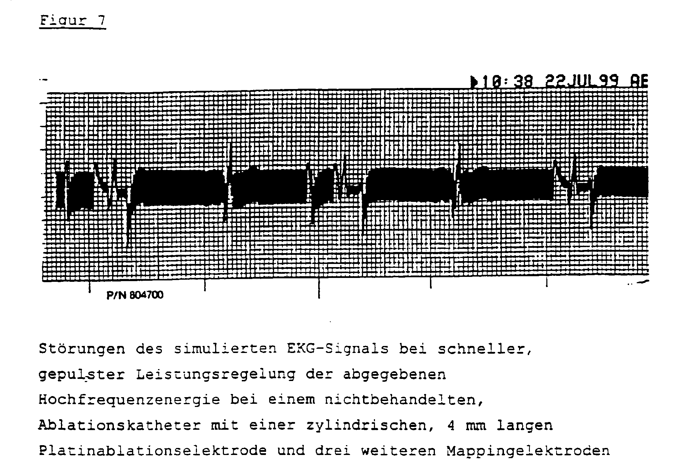

Noch schwieriger ist die Situation bei gepulster Leistungsregelung, wie dies in den Fig. 5 und 7 dargestellt ist, in welchen nahezu überhaupt keine Anteile des EKG-Signals mehr zu erkennen sind.

Die bei diesen Versuchen eingestrahlte Hochfrequenzleistung lag bei etwa 1 bis 50 W, wie es bei der Hochfrequenz-Katheterablation im menschlichen Herzen durchaus üblich ist.

Wurde ein Ablationskatheter jedoch auf die vorstehend beschriebene Weise behandelt, so konnten die überlagerten Störungen bei gleichem Versuchsaufbau sehr stark, jedenfalls um eine Faktor von mehr als zehn reduziert werden, wie es beispielsweise in Figur 3 dargestellt ist.

Der an sich jeweils identische Versuchsaufbau, der sich lediglich darin unterschied, ob der Katheter direkt so, wie er vom jeweiligen Hersteller vertrieben wurde, benutzt wurde oder ob dieser in erfindungsgemäßer Weise behandelt wurde, weist den großen Erfolg der vorliegenden Erfindung unzweideutig nach.

Die erfindungsgemäßen Katheter verfügen folglich an deren Elektrodenoberflächen über weniger elektrische bzw. elektronische Störzentren, welche die überlagerten Signale erzeugen können. Das Maß der Störungsverminderung ist folglich ein Maß für das Vorhandensein bzw. verminderte oder abgeschwächte Vorhandensein derartiger Störzentren.

Zur Erzeugung derartiger, dem EKG-Signal überlagerter, Signale wird ohne Beschränkung der Allgemeinheit und ohne Beschränkung der Erfindung angenommen, daß es zu lokalen Haftstellen bzw. lokalen Extrema der elektrischen Potentiale oder auftretenden Feldstärken an der Oberfläche des Katheters kommt, an welcher Ionen bzw. Moleküle mit Dipolmoment unterschiedlich stark gebunden bzw. bewegt werden können, die dann beim Anlagen der HF-Spannung bzw. -energie durch die gegenüber den gelösten Ionen verschiedene Beweglichkeit ein Spannungssignal erzeugen können, welches sich dem EKG-Signal überlagert.

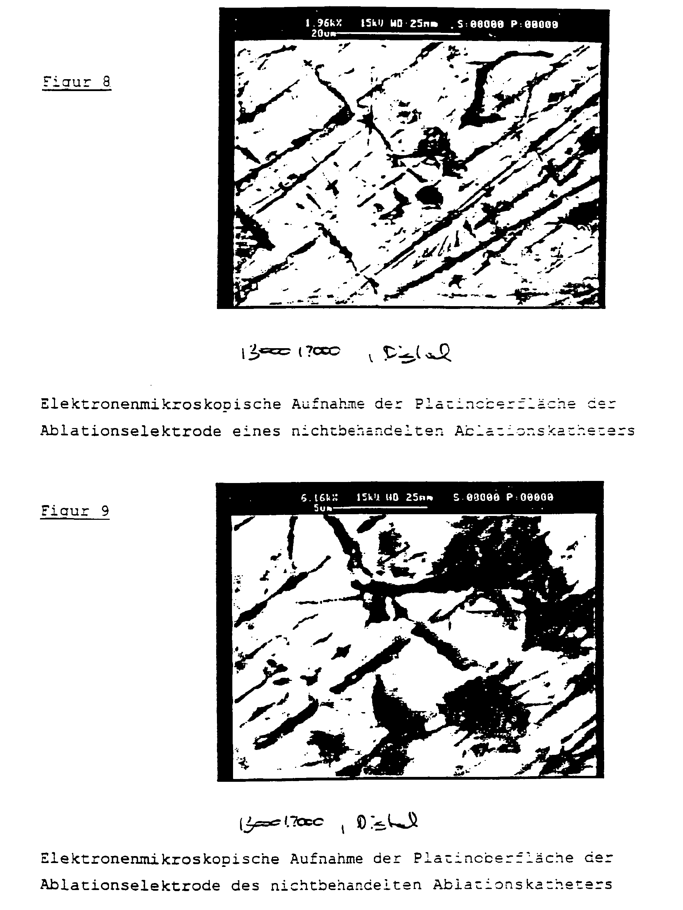

Zum Nachweis eines derartigen Verhaltens wurden die in den Figuren 8 und 9 dargestellten elektronenmikroskopischen Aufnahmen gewonnen, welche zeigen, daß eine im Mikrostrukturbereich scharfkantige Katheteroberfläche vorliegt, welche nach der mechanischen Behandlung weiche Verrundungen und weniger scharfe Riefen oder Rillen aufweist.

Ferner zeigt die AFM- (atomic force microscopic) bzw. kraftmikroskopische Aufzeichnung einer 10 mal 10 µm großen Oberfläche, daß auch elektrische Störzentren an Oberfläche der zumindest einen Ablations- oder Mappingelektrode des Katheters 2 mit zapfen- oder nadelförmiger Gestalt und einem Durchmesser von weniger als 10 µm, insbesondere von weniger als 2 µm vorhanden sind, welche durch das erfindungsgemäße Verfahren in deren Anzahl und Höhe vermindert werden.

Allein durch die mechanische Glättung kann die mechanische Reibung der Ionen an der Oberfläche reduziert werden, so daß hierdurch bedingte mechanisch bewirkte jedoch elektrisch wirksame Störzentren vermindert werden.

Ferner wird angenommen, daß durch die mechanische Behanldung auch elementares oder amorphes, aus dem metallischen kristallinen Metallverbund herausgetrenntes Platin vorliegt. Durch die kinetische Energie und/oder die reine mechanische Trennwirkung des Schleif- oder Poliermittels kann es zum Ablösen von Platinatomen aus dem metallischen kristallinen Verbund und deren amorpher Umlagerung kommen.

Auch eine virtuelle Ablösung, d.h. eine Migration im gebundenen Zustand des Platinatoms führt zu einer Loslösung des Atoms aus dem Kristallverbund und dessen Umlagerung.

Hierdurch lassen sich auch die einem erhöhten Angriff ausgesetzten verrundeten Spitzen der behandelten Oberfläche erklären, denn gerade in diesen Bereichen kann ein Angriff von mehreren Seiten her erfolgen.

Durch diese Migration von Platinatomen können auch an der Oberfläche befindliche Potentiale, etwa an Korngrenzen, oder lokale Feldstärkemaxima an Spitzen oder scharfen Kanten kompensiert werden, so daß auch die effektive elektrische Wirkung derartiger Potentiale oder Feldstärkemaxima drastisch reduziert werden kann.

Somit sind die vor der Behandlung vorliegenden elektrischen Störzentren nicht nur in deren flächiger Erstreckung sondern auch in deren elektrischer Wirkung vermindert.

Die Erfinder fanden auch heraus, daß in vielen Fällen bei einem behandelten Katheter Strukturen der Oberfläche der Ablations- oder Mappingelektrode keine scharfen Kanten, d.h. keine sehr kleine Krümmungsradien mehr aufweisen. In einem Oberflächenabschnitt mit einer Länge, Breite oder Höhe von weniger als 10 µm hatten die vorhandenen Kanten einen Radius von mehr als ungefähr 500 nm.

Schärfere Kanten bzw. kleinere Radien sind in deren Anzahl regelmäßig entweder vermindert oder treten gar nicht mehr auf. Gemäß der Erfindung sollen die Krümmungsradien der meisten auftretenden Kanten mehr als ungefähr 500 nm, vorzugsweise mehr als 100 nm aber wenigstens mehr als 10 nm betragen.

Es sei deutlich darauf hingewiesen, daß erfindungsgemäß behandelte Katheter auch bei nicht angelegter Hochfrequenzenergie eine deutlich verbesserte Signalqualität, d.h. wesentlich geringere Störsignale aufweisen. Diese Verbesserung beschränkt sich nicht auf Ablationselektroden sondern kann auch bei Mapping-Elektroden oder -Kathtetern erfolgreich genutzt werden.

## Patentansprüche

1. Kathether zur Ablation von biologischem, insbesondere von tierischem oder menschlichem Gewebe, vorzugsweise zur Ablation von myokardialem Gewebe des Menschen, mit mindestens einer Ablations- oder Mappingelektrode
**dadurch gekennzeichnet, daß** Strukturen der Oberfläche der mindestens einen Ablations- oder Mappingelektrode eine verrundete Oberflächenstruktur aufweisen, deren Kanten oder Spitzen im Mikrostrukurbereich keine scharfen Kanten, dass heißt einen Krümmungsradius von mehr als 10 nm aufweisen.

2. Katheter nach Anspruch 1, **dadurch gekennzeichnet, daß** die zumindest eine Ablations- oder Mappingelektrode eine mechanisch behandelte Oberfläche aufweist.

3. Katheter nach Anspruch 1 oder 2, **dadurch gekennzeichnet, daß** die zumindest eine Ablations- oder Mappingelektrode eine mechanisch behandelte Oberfläche aufweist, die mit einem die Oberfläche glättenden Verfahren behandelt ist.

4. Katheter nach einem der vorstehenden Ansprüche, **dadurch gekennzeichnet, daß** Strukturen der Oberfläche der mindestens einen Ablations- oder Mappingelektroce eine verrundete Oberflächenstruktur aufweisen, deren Kanten oder Spitzen einen Krümmungsradius von mehr als 100 rm, vorzugsweise von mehr als 500 aufweisen.

5. Katheter nach einem der vorstehenden Ansprüche, **dadurch gekennzeichnet, daß** die zumindest eine Ablations- oder Mappingelektrode ein Edelmetall, insbesondere Platin umfaßt, dessen Oberfläche geglättet ist.

6. Katheter nach einem der vorstehenden Ansprüche, **dadurch gekennzeichnet, daß** die Ablations- oder Mappingelektrodenoberfläche geläppt oder poliert ist.

7. Katheter nach Anspruch 6, **dadurch gekennzeichnet, daß** die Ablations- oder Mappingelektrodenoberfläche mit einem Schleif- oder Poliermittel poliert ist, welches Karbonate, bevorzugt Alkali- oder Erdalkalikarbonate, insbesondere Kalziumkarbonat enthält.

8. Katheter nach einem der vorstehenden Ansprüche, **dadurch gekennzeichnet, daß** die Ablations- oder Mappingelektrodenoberfläche mit einer glatten Walze gewälzt ist.

9. Verfahren zur Herstellung eines Katheters zur Ablation von myokardialem Gewebe gemäß einem der vorstehenden Ansprüche,
wobei
die zumindest eine Ablations- oder Mappingelektrode an deren Oberfläche behandelt wird, und
die zu behandelnde Ablations- oder Mappingelektrode des Katheters geschliffen, geläppt und/oder poliert wird.

10. Verfahren zur Herstellung eines Katheters nach Anspruch 9, bei welchem die Oberfläche der zumindest einen Ablations- oder Mappingelektrode des Katheters mit einer glatten Walze gewalzt wird.

11. Verfahren zur Herstellung eines Katheters nach einem der vorstehenden Ansprüche, bei welchem die Oberfläche der zumindest einen Ablations- oder Mappingelektrode des Katheters mit einem weichen Schleif- oder Poliermittel behandelt wird, welches ein Karbonat, vorzugsweise ein Alkali- oder Erdalkalikarbonat umfasst.

12. Verfahren zur Herstellung eines Katheters nach einem der vorstehenden Ansprüche, bei welchem Strukturen an der Oberfläche der zumindest einen Ablations- oder Mappingelektrode des Katheters mit Krümmungsradien von mehr als 10 nm, vorzugsweise von mehr als 100 nm aber mehr als 500 nm erzeugt werden.

13. Verfahren zur Herstellung eines Katheters nach einem der vorstehenden Ansprüche, bei welchem Strukturen an der Oberfläche der zumindest einen Ablations- oder Mappingelektrode des Katheters mit nadelförmiger Gestalt und einem Durchmesser von weniger als 10 µm in deren Anzahl vermindert werden.

14. Verfahren zur Herstellung eines Katheters nach einem der vorstehenden Ansprüche, bei welchem die mechanische Bearbeitung das Auftragen einer Folie mit glatter Oberfläche, insbesondere das Aufreiben der Folie mit glatter Oberfläche auf die zumindest eine Ablations- oder Mappingelektrode umfasst.

## Claims

1. Catheter for ablation of biological tissue, particularly human or animal, preferably for the ablation of myocardial tissue of a human, with at least one ablation or mapping electrode, **characterised in that** structures on the surface of the at least one ablation or mapping electrode have a rounded surface structure, of which the edges or tips do not have any sharp edges in the microstructure range, that is to say have a radius of curvature of more than 10 nm.

2. Catheter according to claim 1, **characterised in that** the at least one ablation or mapping electrode has a mechanically treated surface.

3. Catheter according to claim 1 or 2, **characterised in that** the at least one ablation or mapping electrode has a mechanically treated surface which is treated with a surface smoothing process.

4. Catheter according to one of the preceding claims, **characterised in that** structures on the surface of the at least one ablation or mapping electrode have a rounded surface structure, of which the edges or tips have a radius of curvature of more than 100 rm, preferably more than 500.

5. Catheter according to one of the preceding claims, **characterised in that** the at least one ablation or mapping electrode comprises a noble metal, in particular platinum, of which the surface is smoothed.

6. Catheter according to one of the preceding claims, **characterised in that** the ablation or mapping electrode surface is lapped or polished.

7. Catheter according to clam 6, **characterised in that** the ablation or mapping electrode surface is polished with a grinding or polishing agent which contains carbonate, preferably alkaline or earth alkaline carbonate, in particular calcium carbonate.

8. Catheter according to one of the preceding claims, **characterised in that** the ablation or mapping electrode surface is rolled with a smooth roller.

9. Method for producing a catheter for ablation of myocardial tissue according to one of the preceding claims, wherein
the at least one ablation or mapping electrode is treated on its surface, and
the ablation or mapping electrode of the catheter to be treated is ground, lapped and / or polished.

10. Method for producing a catheter according to claim 9, wherein the surface of the at least one ablation or mapping electrode of the catheter is rolled with a smooth roller.

11. Method for producing a catheter according to one of the preceding claims, wherein the surface of the at least one ablation or mapping electrode of the catheter is treated with a soft grinding or polishing agent which comprises a carbonate, preferably an alkaline or earth alkaline carbonate.

12. Method for producing a catheter according to one of the preceding claims, wherein structures on the surface of the at least one ablation or mapping electrode of the catheter are produced with radii of curvature of more than 10 nm, preferably more than 100 nm but more than 500 nm.

13. Method for producing a catheter according to one of the preceding claims, wherein structures on the surface of the at least one ablation or mapping electrode of the catheter with a needle-like form and a diameter of less than 10 µm are reduced in number.

14. Method for producing a catheter according to one of the preceding claims, wherein the mechanical processing comprises the application of a film with a smooth surface, in particular the rubbing of the film with a smooth surface onto the at least one ablation or mapping electrode.

## Revendications

1. Cathéter pour l'ablation de tissu biologique, en particulier de tissu animal ou humain, de préférence pour l'ablation de tissu myocardique de l'homme, comprenant au moins une électrode d'ablation ou une électrode de cartographie, **caractérisé en ce que** des structures de la surface de la au moins une électrode d'ablation ou électrode de cartographie présente une structure de surface arrondie, dont les bords ou pointes ne présentent pas d'arêtes tranchantes dans la zone de microstructure, c'est-à-dire un rayon de courbure de plus de 10 nm.

2. Cathéter selon la revendication 1, **caractérisé en ce que** la au moins une électrode d'ablation ou électrode de cartographie présente une surface traitée mécaniquement.

3. Cathéter selon la revendication 1 ou 2, **caractérisé en ce que** la au moins une électrode d'ablation ou de cartographie présente une surface traitée mécaniquement qui est traitée avec un procédé lissant la surface.

4. Cathéter selon l'une quelconque des revendications précédentes, **caractérisé en ce que** des structures de la surface de la au moins une électrode d'ablation ou de cartographie présente une structure de surface arrondie, dont les arêtes ou pointes présentent un rayon de courbure de plus de 100 rm, de préférence de plus de 500.

5. Cathéter selon l'une quelconque des revendications précédentes, **caractérisé en ce que** la au moins une électrode d'ablation ou électrode de cartographie comprend un métal noble, en particulier du platine, dont la surface est lissée.

6. Cathéter selon l'une quelconque des revendications précédentes, **caractérisé en ce que** la surface de l'électrode d'ablation ou de l'électrode de cartographie est rodée ou polie.

7. Cathéter selon la revendication 6, **caractérisé en ce que** la surface de l'électrode d'ablation ou de l'électrode de cartographie est polie avec un agent abrasif ou de polissage qui contient des carbonates et de préférence des carbonates alcalins ou des carbonates alcalino-terreux, en particulier du carbonate de calcium.

8. Cathéter selon l'une quelconque des revendications précédentes, **caractérisé en ce que** la surface de l'électrode d'ablation ou de l'électrode de cartographie est roulée avec un rouleau lisse.

9. Procédé pour la fabrication d'un cathéter pour l'ablation de tissu myocardique selon l'une quelconque des revendications précédentes,
la au moins une électrode d'ablation ou électrode de cartographie étant traitée sur sa surface et
l'électrode d'ablation ou de cartographie à traiter du cathéter étant rectifiée, rodée et/ou polie.

10. Procédé pour la fabrication d'un cathéter selon la revendication 9, dans lequel la surface de la au moins une électrode d'ablation ou de cartographie du cathéter est roulée avec un rouleau lisse.

11. Procédé pour la fabrication d'un cathéter selon l'une quelconque des revendications précédentes, dans lequel la surface de la au moins une électrode d'ablation ou de cartographie du cathéter est traité avec un agent abrasif ou de polissage souple, qui comprend un carbonate, de préférence un carbonate alcalin ou un carbonate alcalino-terreux.

12. Procédé pour la fabrication d'un cathéter selon quelconque des revendications précédentes, dans lequel des structures sur la surface de la au moins une électrode d'ablation ou électrode de cartographie du cathéter sont générées avec des rayons de courbure de plus de 10 nm, de préférence de plus de 100 nm, mais plus de 500 nm.

13. Procédé pour la fabrication d'un cathéter selon quelconque des revendications précédentes, dans lequel des structures sur la surface de la au moins une électrode d'ablation ou électrode de cartographie du cathéter avec contour aciculaire et un diamètre de moins de 10 µm sont réduits dans leur nombre.

14. Procédé pour la fabrication d'un cathéter selon quelconque des revendications précédentes, dans lequel l'usinage mécanique comprend l'application d'un film avec une surface lisse, en particulier l'alésage du film avec une surface lisse sur la au moins une électrode d'ablation ou de cartographie.
